# EUROPEAN PATENT SPECIFICATION

(11) **EP 2 595 604 B1**
(45) Date of publication and mention of the grant of the patent: **30.03.2016**
(21) Application number: 11741503.4
(22) Date of filing: 20.07.2011
(51) Int. Cl.: A61K 9/00, A61K 47/10, A61K 47/26, A61K 47/32, A61K 47/36, A61K 47/38, A61K 47/40, A61K 48/00

(54) **METHOD OF CONTROLLING INITIAL DRUG RELEASE OF SIRNA FROM SUSTAINED-RELEASE IMPLANTS**
VERFAHREN ZUR STEUERUNG DER ANFÄNGLICHEN WIRKSTOFFFREISETZUNG VON SIRNA AUS RETARD-IMPLANTATEN
PROCÉDÉ DE CONTRÔLE DE LA LIBÉRATION INITIALE DE MÉDICAMENT D'ARNSI PAR DES IMPLANTS À LIBÉRATION RETARDÉE

(30) Priority: 21.07.2010 US 366504 P
(43) Date of publication of application: 29.05.2013
(73) Proprietor: ALLERGAN, INC., Irvine, CA 92612 (US)
(72) Inventor: RIVERS, Hongwen M., San Diego, California 92127 (US); SPADA, Lon T., Walnut, California 91789 (US); LUU, Michelle, Anaheim, California 92802 (US)
(74) Representative: Hoffmann Eitle
(86) International application number: PCT/US2011/044701
(87) International publication number: WO 2012/012546

(56) References cited:
- CA-A1- 2 621 055
- US-A1- 2008 107 694
- US-A1- 2009 258 924

## Description

### BACKGROUND OF THE INVENTION

The present invention relates to ocular implants comprising siRNA combined with a pharmaceutical excipient effective to retard the initial release of siRNA , the combination being associated with a biocompatible polymer and configured to release said siRNA into the eye of a patient at therapeutic levels for a time sufficient to treat an ocular condition or disease, wherein the high initial drug release (burst) due to the water solubility of siRNA is retarded.

Small interfering RNA (siRNA), sometimes known as short interfering RNA or silencing RNA, is a class of double-stranded RNA molecules, 20-25 nucleotides (nt) in length, that play a variety of roles in biology. siRNA is involved in the RNA interference (RNAi) pathway, where it interferes with the expression of a specific gene.

Synthetic siRNAs have been shown to be able to induce RNAi in mammalian cells. This discovery has led to a surge in interest in harnessing RNAi for biomedical research and drug development.

siRNAs have a well-defined structure: a short (usually 21-nt) double strand of RNA (dsRNA) with 2-nt 3' overhangs on either end:

Each strand has a 5' phosphate group and a 3' hydroxyl (-OH) group. This structure is the result of processing by dicer, an enzyme that converts either long dsRNAs or small hairpin RNAs into siRNAs. siRNAs can also be exogenously (artificially) introduced into cells by various transfection methods to bring about the specific knockdown of a gene of interest. Essentially any gene of which the sequence is known can thus be targeted based on sequence complementarily with an appropriately tailored siRNA. This has made siRNAs an important tool for gene function and drug target validation studies in the post-genomic era.

Transfection of an exogenous siRNA can be problematic because the gene knockdown effect is only transient, particularly in rapidly dividing cells. One way of overcoming this challenge is to modify the siRNA in such a way as to allow it to be expressed by an appropriate vector, e.g., a plasmid. This is done by the introduction of a loop between the two strands, thus producing a single transcript, which can be processed into a functional siRNA. Such transcription cassettes typically use an RNA polymerase III promoter (e.g., U6 or H1), which usually directs the transcription of small nuclear RNAs (snRNAs) (U6 is involved in gene splicing; H1 is the RNase component of human RNase P). It is assumed (although not known for certain) that the resulting siRNA transcript is then processed by Dicer.

It has been found that dsRNA can also activate gene expression, a mechanism that has been termed "small RNA-induced gene activation" or RNAa. It has been shown that dsRNAs targeting gene promoters induce potent transcriptional activation of associated genes. RNAa was demonstrated in human cells using synthetic dsRNAs, termed "small activating RNAs"

### (saRNAs).

Given the ability to knock down essentially any gene of interest, RNAi via siRNAs has generated a great deal of interest in both basic and applied biology. There are an increasing number of large-scale RNAi screens that are designed to identify the important genes in various biological pathways. Because disease processes also depend on the activity of multiple genes, it is expected that in some situations turning off the activity of a gene with a siRNA will produce a therapeutic benefit.

Results of therapeutic RNAi trials indicated for age-related macular degeneration, (AMD) demonstrated that siRNAs are well tolerated and have suitable pharmacokinetic properties. siRNAs and related RNAi induction methods therefore stand to become an important new class of drugs in the foreseeable future.

Despite the potential benefits of developing drugs based on siRNAs, positively charged and highly water soluble siRNAs are known to be difficult to formulate into sustained release implants because their high water solubility and anionic nature leads to high initial rates of release when the implant is implanted into the eye of a patient.

### BRIEF SUMMARY OF THE INVENTION

The present invention is defined in the claims and provides an ocular implant comprising siRNA combined with a excipient effective to retard the initial burst release of the siRNA from an implant, wherein said siRNA and excipient is associated with a biocompatible polymer. e.g. a polymeric matrix, configured to release said siRNA into the eye of a patient at therapeutic levels for a time sufficient to treat an ocular condition or disease.

### BRIEF DESCRIPTION OF THE DRAWINGS

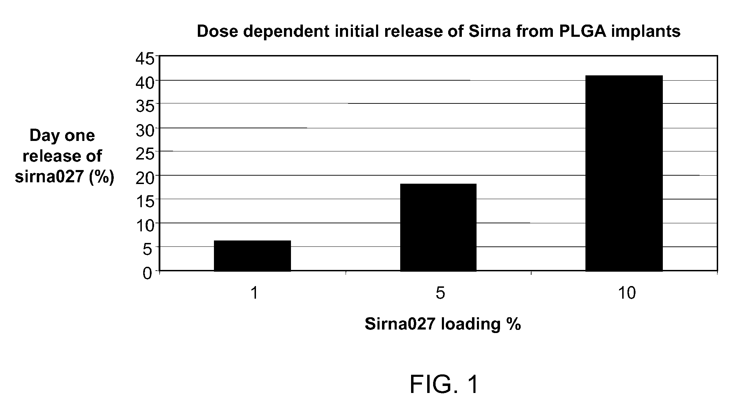

Figure 1 shows the effect of siRNA loading on the initial burst of siRNA from PLGA implants. The amount of siRNA loaded into each implant is shown on the x-axis in the graph as the percent siRNA by weight of PLGA polymer. An *in vitro* release study was conducted and the amount of siRNA released within one day was measured as initial burst.

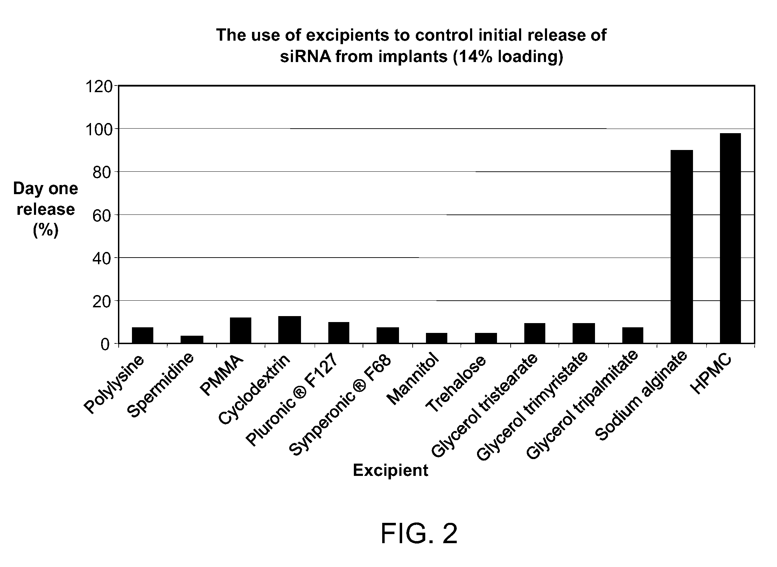

Figure 2 shows the effect of certain excipients on the initial burst of siRNA released from biodegradable implants. The excipients tested are shown on the x-axis. Each formulation contained 5% (w/w) excipient, 14% (w/w) siRNA, and 81% (w/w) biodegradable PLGA polymer (RG752S).

### DETAILED DESCRIPTION OF THE INVENTION

### Definitions

The following terms as used herein have the following meanings:

The term "Initial burst release of siRNA" from an implant, also referred to herein as "the initial rate of release" of an siRNA from an implant, refers to the amount of siRNA released from an implant in one day (24 hours) after placement in the eye of a subject. The initial burst release, or the amount of siRNA released from an implant during a given period of time, such as after 24 hr in the eye, may be expressed as a percentage of the amount of siRNA initially loaded in the implant. Experimentally, the effects of different excipients on the "Initial burst release of siRNA" from an implant can be evaluated using an *in vitro* release assay (see Example 1, for example), whereby an siRNA-containing implant is incubated in a buffered solution (e.g., phosphate buffered saline, PBS) at physiological pH and temperature.

The term "sustained release" means release of an active agent, such as an siRNA, over a period of at least about seven days or more.

The term "extended release" means release of an active agent, such as an siRNA, over a period of at least about one day or more.

The term "individual" means a human person, subject, or patient.

"Biodegradable polymer" means a polymer or polymers which degrade in vivo, and wherein erosion of the polymer or polymers over time occurs concurrently with or subsequent to release of the therapeutic agent. Specifically, hydrogels such as methylcellulose which act to release drug through polymer swelling are specifically excluded from the term "biodegradable polymer". The terms "biodegradable" and "bioerodible" are equivalent and are used interchangeably herein. A biodegradable polymer may be a homopolymer, a copolymer, or a polymer comprising more than two different polymeric units.

"Ocular condition" means a disease, ailment or pathological condition which affects or involves the eye or one of the parts or regions of the eye. Broadly speaking the eye includes the eyeball and the tissues and fluids which constitute the eyeball, the periocular muscles (such as the oblique and rectus muscles) and the portion of the optic nerve which is within or adjacent to the eyeball.

"Intraocular implant" means a device or element that is structured, sized, or otherwise configured to be placed "in an eye" of a living mammal, including the subconjunctival space. Intraocular implants are generally biocompatible with physiological conditions of an eye and do not cause adverse side effects. Intraocular implants may be placed in an eye without disrupting vision of the eye.

The term "biocompatible" means compatible with living tissue or a living system by not being toxic, injurious, or physiologically reactive and not causing an immunological reaction.

The terms "treat", "treating", or "treatment" mean a reduction or resolution or prevention of an ocular condition, ocular injury or damage, or to promote healing of injured or damaged ocular tissue.

"Therapeutically effective amount" means the level or amount of agent needed to treat an ocular condition, or reduce or prevent ocular injury or damage without causing significant negative or adverse side effects to the eye or a region of the eye.

The "initial load of siRNA" in an implant is the mass of siRNA present in the implant before placement in the eye. The mass of siRNA or any other component in the implant, may be expressed as a percentage of the total mass of the implant, i.e., as the percent by weight of siRNA in the implant (% w/w).

As used herein, the "percent by weight" of a component (such as an siRNA, biodegradable polymer, or excipient) present in an implant is equivalent to and may be used interchangeably with the "weight percent" of a component in an implant to express the amount a component present in a biodegradable intraocular implant of the invention. The "percent by weight" and "weight percent" of a component present in an implant is defined herein as the (weight (or mass) of component ÷ the total weight (or mass) of the implant) x 100.

"PLGA" is a polylactic acid polyglycolic acid copolymer, and is sometimes referred to as a poly(lactide-co-glycolide) or poly(lactic-co-glycolic acid). More specifically, the PLGA used in an implant may be a poly(D,L-lactide-co-glycolide).

"PLA" is a polylactic acid and is referred to as polylactide. More specifically, the PLA used in an implant may be a poly(D,L-lactide).

The present invention is defined in the claims as a biodegrable intraocular implant for the extended release of a siRNA, the implant comprising one or more biodegradable polymers, an siRNA, and an excipient, wherein the one or more biodegradable polymers is selected from the group consisting of polylactic acid, polyglycolic acid, polylactic acid polyglycolic acid copolymer (PLGA), and mixtures thereof, wherein the excipient is selected from polylysine and spermidine, whereby the implant provides extended release of the siRNA in an eye of an individual when placed in the eye of an individual.

The excipient decreases or retards the initial rate of release of the siRNA from the implant. The siRNA is associated with the biocompatible polymer e.g. a polymeric matrix. The implant is configured to release said siRNA into the eye of a patient at therapeutic levels for a time sufficient to treat an ocular condition or disease.

The excipient is selected from polylysine and spermidine, which are molecules that can form a combination with siRNAs based on their charge-charge interactions.

These excipients are first dissolved in water and mixed with an aqueous solution of siRNA before they are lyophilized into a dry powder. The dry powder mixture of siRNA and excipient is then blended with PLA/PLGA polymers, and compacted into pellets, or hot melt extruded into solid implants.

Notwithstanding the high water solubility of siRNAs which makes it difficult to produce highly loaded implants necessary for a sustained duration of action, the resulting implants can have a higher loading making it possible to achieve a longer-duration sustained release of the siRNA.

siRNAs are well known in the art and may be identified in accordance with procedures disclosed in various publications, including, for example, US Patent Number 7,700,760. Thus, the siRNA utilized in the compositions and methods of this invention are those effective to treat diseases and conditions of the eye, i.e. ophthalmic diseases and conditions.

For example, implants of the present invention may include one or more siRNAs directed to the gene encoding vascular endothelial growth factor (VEGF).

The combined siRNA and the excipient are formed into an implant by associating such components with a polymer that is compatible with the body of the patient, e.g. an ocular implant will comprise a polymer that is compatible with the environment of the eye of the patient being treated by the implantation of the implant of this invention.

Suitable polymeric materials or compositions for use in the implant include those materials which are compatible, i.e. biocompatible, with the eye so as to cause no substantial interference with the functioning or physiology of the eye. Such materials are biodegradable or bioerodible.

Examples of useful polymeric materials include, without limitation, such materials derived from and/or including organic esters and organic ethers, which when degraded result in physiologically acceptable degradation products, including the monomers. Also, polymeric materials derived from and/or including, anhydrides, amides, orthoesters and the like, by themselves or in combination with other monomers, may also find use. The polymeric materials may be addition or condensation polymers, advantageously condensation polymers. The polymeric materials may be cross-linked or non-cross-linked, for example not more than lightly cross-linked, such as less than about 5%, or less than about 1% of the polymeric material being cross-linked. For the most part, besides carbon and hydrogen, the polymers will include at least one of oxygen and nitrogen, advantageously oxygen. The oxygen may be present as oxy, e.g. hydroxy or ether, carbonyl, e.g. non-oxo-carbonyl, such as carboxylic acid ester, and the like. The nitrogen may be present as amide, cyano and amino. The polymers set forth in Heller, Biodegradable Polymers in Controlled Drug Delivery, In: CRC Critical Reviews in Therapeutic Drug Carrier Systems, Vol. 1, CRC Press, Boca Raton, Fla. 1987, pp 39-90, which describes encapsulation for controlled drug delivery, may find use in the present implants.

Of additional interest are polymers of hydroxyaliphatic carboxylic acids, either homopolymers or copolymers, and polysaccharides. Polyesters of interest include polymers of D-lactic acid, L-lactic acid, racemic lactic acid, glycolic acid, polycaprolactone, and combinations thereof. Generally, by employing the L-lactate or D-lactate, a slowly eroding polymer or polymeric material is achieved, while erosion is substantially enhanced with the lactate racemate.

Among the useful polysaccharides are, without limitation, calcium alginate, and functionalized celluloses, particularly carboxymethylcellulose esters characterized by being water insoluble, a molecular weight of about 5 kD to 500 kD, for example.

Other polymers of interest include, without limitation, polyvinyl alcohol, polyesters, polyethers and combinations thereof which are biocompatible and may be biodegradable and/or bioerodible.

Some preferred characteristics of the polymers or polymeric materials for use in the present invention may include biocompatibility, compatibility with the siRNA and/or the siRNA combined, ease of use of the polymer in making the implant of the present invention, a half-life in the physiological environment of at least about 6 hours, preferably greater than about one day, and not significantly increasing the viscosity of the vitreous.

The biodegradable polymeric materials which are included to form the matrix are desirably subject to enzymatic or hydrolytic instability. Water-soluble polymers may be cross-linked with hydrolytic or biodegradable unstable cross-links to provide useful water insoluble polymers. The degree of stability can be varied widely, depending upon the choice of monomer, whether a homopolymer or copolymer is employed, employing mixtures of polymers, and whether the polymer includes terminal acid groups.

Equally important to controlling the biodegradation of the polymer and hence the extended release profile of the implant is the relative average molecular weight of the polymeric composition employed in the implant. Different molecular weights of the same or different polymeric compositions may be included in the implant to modulate the release profile. In certain implants, the relative average molecular weight of the polymer will range from about 9 to about 64 kD, usually from about 10 to about 54 kD, and more usually from about 12 to about 45 kD.

In some implants according to the invention, copolymers of glycolic acid and lactic acid are used, referred to as poly(lactide-co-glycolide)s or PLGAs, where the rate of biodegradation is controlled by the ratio of glycolic acid to lactic acid. The most rapidly degraded poly(lactide-co-glycolide) (PLGA) copolymer has roughly equal amounts of glycolic acid and lactic acid. Homopolymers, or copolymers having ratios other than equal, are more resistant to degradation. The ratio of glycolic acid to lactic acid will also affect the brittleness of the implant, where a more flexible implant is desirable for larger geometries. The percent of polylactic acid in the poly(lactide-co-glycolide) (PLGA) copolymer can be 0-100%, preferably about 15-85%, more preferably about 35-65%. In some implants, a 50/50 PLGA copolymer is used.

The biodegradable polymer matrix of the intraocular implant may comprise a mixture of one or two or more biodegradable polymers. For example, the implant may comprise a mixture of a first biodegradable polymer and a different second biodegradable polymer. One or more of the biodegradable polymers may have terminal acid groups.

Release of a drug from an erodible polymer is the consequence of several mechanisms or combinations of mechanisms. Some of these mechanisms include desorption from the implant's surface, dissolution, diffusion through porous channels of the hydrated polymer and erosion. Erosion can be bulk or surface or a combination of both. As discussed herein, the matrix of the intraocular implant may release drug at a rate effective to sustain release of an amount of siRNA for more than one week after implantation into an eye. In certain implants, therapeutic amounts of siRNA are released for no more than about 30-35 days after implantation. For example, an implant may comprise siRNA, and the matrix of the implant degrades at a rate effective to sustain release of a therapeutically effective amount of siRNA for about one month after being placed in an eye. As another example, the implant may comprise siRNA, and the matrix releases drug at a rate effective to sustain release of a therapeutically effective amount of siRNA for more than forty days, such as for about six months.

In one embodiment, the implant may be provided in the form of a rod or a filament produced by an extrusion process.

An intraocular implant formulation within the scope of this disclosure can comprise, consist of, or consist essentially of 30% by weight (w/w) siRNA, 45% by weight R203S (a poly (D,L-lactide)), 20% by weight R202H, and 5% by weight PEG 3350; or 20% by weight siRNA, 45% by weight R203S, 10% by weight R202H, 20% weight RG752S (a 75:25 poly (D,L lactide-co-glycolide)), and 5% by weight PEG 3350. The range of concentrations of the constituents that can be used in the preferred implant formulation are siRNA 5 to 40% by weight, R203S 10 to 60% by weight, R202H 5 to 20% by weight, RG752S 5 to 40% by weight, and PEG 3350 0 to 15% by weight. The PLA/PLGA polymers are from the Resomer product line available from Evonik Industries, Germany, and include the listing in Table 1.

**Table 1**

| **Resomer** | **Monomer ratio** | **inherent viscosity dL/g** |
|---|---|---|
| RG502, | 50:50 poly (D, L-lactide-co-glycolide) | 0.2 |
| RG502H, | 50:50 poly (D, L-lactide-co-glycolide) | 0.2 |
| RG503, | 50:50 poly (D, L-lactide-co-glycolide) | 0.3 |
| RG504, | | 0.4 |
| RG505, | | 0.5 |
| RG506, | | 0.6 |
| RG752, | 75:25 poly (D,L lactide-co-glycolide) | 0.2 |
| RG755, | 75:25 poly(D,L lactide-co-glycolide) | 0.5 (40000) |
| RG756, | | 0.6 |
| RG858, | 85:15 poly (D,L-lactide-co-glycolide) | 1.0 |
| R202H, | poly (D,L-lactide) | 0.2 |
| R203 | poly (D,L-lactide) | 0.3(40000) |
| R206. | poly (D,L-lactide) ; acid end | 0.6 |
| R104 | poly (D,L-lactide) | (3500) |

The release of the siRNA from the intraocular implant comprising a biodegradable polymer matrix may include an initial burst of release followed by a gradual increase in the amount of the siRNA released, or the release may include an initial delay in release of the siRNA component followed by an increase in release. When the implant is substantially completely degraded, the percent of the siRNA that has been released is about one hundred.

It may be desirable to provide a relatively constant rate of release of the siRNA from the implant over the life of the implant. The release profile of the siRNA may include one or more linear portions and/or one or more non-linear portions. Preferably, the release rate is greater than zero once the implant has begun to degrade or erode.

The implants may be monolithic, i.e. having the active agent or agents homogenously distributed through the polymeric matrix, or encapsulated, where a reservoir of active agent is encapsulated by the polymeric matrix. Due to ease of manufacture, monolithic implants are usually preferred over encapsulated forms. However, the greater control afforded by the encapsulated, reservoir-type implant may be of benefit in some circumstances, where the therapeutic level of the siRNA falls within a narrow window. Alternatively, or in addition, the siRNA, may be distributed in a non-homogenous pattern in the matrix. For example, the implant may include a portion that has a greater concentration of the siRNA relative to a second portion of the implant.

The intraocular implants disclosed herein may have a size of between about 5 µm and about 10 mm, or between about 10 µm and about 1 mm for administration with a needle, greater than 1 mm, or greater than 2 mm, such as 3 mm or up to 10 mm, for administration by surgical implantation. For needle-injected implants, the implants may have any appropriate length so long as the diameter of the implant permits the implant to move through a needle. For example, implants having a length of about 6 mm to about 7 mm have been injected into an eye. The implants administered by way of a needle should have a diameter that is less than the inner diameter of the needle. In certain implants, the diameter is less than about 500 µm. The vitreous chamber in humans is able to accommodate relatively large implants of varying geometries, having lengths of, for example, 1 to 10 mm. For example, humans have a vitreous volume of approximately 3.8 mL. The implant may be a cylindrical pellet (e. g., rod) with dimensions of about 2 mm times 0.75 mm diameter. Or the implant may be a cylindrical pellet with a length of about 7 mm to about 10 mm, and a diameter of about 0.75 mm to about 1.5 mm.

The implants may also be at least somewhat flexible so as to facilitate both insertion of the implant in the eye, such as in the vitreous, and accommodation of the implant. The total weight of the implant is usually about 250-5000 µg, more preferably about 500-1000 µg. For example, an implant may be about 500 µg, or about 1000 µg.

Thus, implants can be prepared where the center may be of one material and the surface may have one or more layers of the same or a different composition, where the layers may be cross-linked, or of a different molecular weight, different density or porosity, or the like. For example, where it is desirable to quickly release an initial bolus of drug, the center may be a polylactate coated with a polylactate-polyglycolate copolymer, so as to enhance the rate of initial degradation. Alternatively, the center may be polyvinyl alcohol coated with polylactate, so that upon degradation of the polylactate exterior the center would dissolve and be, rapidly washed out of the eye.

The implants may be of any geometry including fibers, sheets, films, microspheres, spheres, circular discs, plaques and the like. The upper limit for the implant size will be determined by factors such as toleration for the implant, size limitations on insertion, ease of handling, etc. Where sheets or films are employed, the sheets or films will be in the range of at least about 0.5 mm.times.0.5 mm, usually about 3-10 mm.times.5-10 mm with a thickness of about 0.1-1.0 mm for ease of handling. Where fibers are employed, the fiber diameter will generally be in the range of about 0.05 to 3 mm and the fiber length will generally be in the range of about 0.5-10 mm. Spheres may be in the range of about 0.5 µmto 4 mm in diameter, with comparable volumes for other shaped particles.

The size and form of the implant can also be used to control the rate of release, period of treatment, and siRNA concentration at the site of implantation. Larger implants will deliver a proportionately larger dose, but depending on the surface to mass ratio, may have a slower release rate. The particular size and geometry of the implant are chosen to suit the site of implantation.

The proportions of the siRNA combined, polymer, and any other modifiers may be empirically determined by formulating several implants with varying proportions. A USP approved method for dissolution or release test can be used to measure the rate of release (USP 23; NF 18 (1995) pp. 1790-1798). For example, using the infinite sink method, a weighed sample of the implant is added to a measured volume of a solution containing 0.9% NaCl in water, where the solution volume will be such that the siRNA combined concentration after release is less than 5% of saturation. The mixture is maintained at 37.degree. C. and shaken slowly. The appearance of the dissolved siRNA combined as a function of time may be followed by various methods known in the art, such as spectrophotometrically, HPLC, mass spectroscopy, etc. until the absorbance becomes constant or until greater than 90% of the drug has been released.

The cellular uptake of siRNA can be monitored by blending in a trace amount of fluorescein labeled siRNA and visualizing the localization of siRNA under fluorescence microscope. The gene silencing activities of siRNA released from the implants described here can be analyzed using a cultured mammalian cell assay.

In addition to the siRNA included in the intraocular implants disclosed herein, the intraocular implants may also include one or more additional ophthalmically acceptable therapeutic agents as described in US Patent Application 10/837,260. The intraocular implants disclosed herein may also include effective amounts of buffering agents, preservatives and the like.

In certain implants, an implant comprising siRNA and a biodegradable polymer matrix is able to release or deliver an amount of siRNA between about 0.1 mg to about 0.5 mg for about 3-6 months after implantation into the eye. The implant may be configured as a rod or a wafer. A rod-shaped implant may be derived from filaments extruded from a 720 µm nozzle and cut into 1 mg size. A wafer-shaped implant may be a circular disc having a diameter of about 2.5 mm, a thickness of about 0.127 mm, and a weight of about 1 mg.

The present implants are configured to release an amount of siRNA effective to treat an ocular condition, such as by reducing at least one symptom of the ocular condition. More specifically, the implants may be used in a method to treat any angiogenic disorder like choroidal neovascularization associated with age related macular degeneration.

In one embodiment, an implant, such as the implants disclosed herein, is administered to a posterior segment of an eye of a human patient. In at least one embodiment, an implant is administered without accessing the subretinal space of the eye. For example, a method of treating a patient may include placing the implant directly into the posterior chamber of the eye. In other embodiments, a method of treating a patient may comprise administering an implant to the patient by at least one of intravitreal injection, subconjuctival injection, sub-tenon injections, retrobulbar injection, and suprachoroidal injection.

In addition, for dual therapy approaches to treating an ocular condition, the method may include one or more additional steps of administering additional therapeutic agents to the eye, such as by topically administering compositions containing said other therapeutic agent.

In certain implants according to this disclosure, the implant comprises a therapeutic component which consists essentially of an siRNA and a biodegradable polymer matrix. The biodegradable polymer matrix may comprise or consist essentially of PLA, PLGA, or a combination thereof. When placed in the eye, the implant releases about 10% to about 25% of the siRNA to provide a loading dose of the siRNA within about one day after placement in the eye. Subsequently, the implant releases about 1% to about 2% of the siRNA per day to provide a sustained therapeutic effect.

In implants according to the present invention, the implant comprises, consists of, or consists essentially of an amount of siRNA, a biodegradable polymer matrix, and an excipient. The biodegradable polymer matrix may comprise or consist essentially of polylactic acid (PLA), a polylactic acid polyglycolic acid copolymer (PLGA), or a combination of PLA polymer(s) and PLGA copolymer(s). When placed in the eye, the implant releases between about 2% and about 10% of the siRNA within about one day after placement in the eye. The excipient is selected from polylysine and spermidine.

In another embodiment, an implant according to the present invention comprises, consists of, or consists essentially of a small interfering RNA, a biodegradable polymer or combination of biodegradable polymers, and an excipient, wherein said polymer or polymer combination is selected from the group consisting of polylactic acid (PLA), polglycolic acid (PGA), polylactic acid polyglycolic acid (PLGA) copolymer, and any combinations thereof, and wherein said excipient retards the rate of release of siRNA from the implant relative to an implant consisting essentially of the siRNA and the biodegradable polymer or polymer combination, and is selected from polylysine and spermidine.

An implant of the present invention, including any of those described herein above, can comprise from about 1 to about 40% (w/w) siRNA, about 5 to about 35% (w/w/) siRNA, about 10 to about 30% (w/w) siRNA, about 15 to about 25% (w/w) siRNA, or about 20% (w/w) siRNA. In more specific embodiments, the implant of the present invention can comprise about 1, 2, 3, 4, 5, 6, 7, 8, 9, 10, 11, 12, 13, 14, 15, 16, 17 , 18 , 19, 20, 21, 22, 23, 24, 25, 26, 27, 28 , 29, 30, 31, 32, 33, 34, 35, 36, 37, 38 , 39, or about 40% (w/w) siRNA.

In addition to siRNA, an implant of the present invention, including any of those described herein above, preferably comprises from about 10 to about 90% by weight of a biodegradable polymer or combination of biodegradable polymers. More specifically, an implant of the present invention may comprise from about 15 to about 85% by weight of a polymer or polymer combination, from about 20 to about 80% by weight (w/w), from about 25 to about 75% (w/w), from about 30 to about 70% (w/w), from about 35 to about 65% (w/w), from about 40 to about 60% (w/w), from about 45 to about 55% (w/w), or about 50% (w/w) of a polymer or polymer combination. Even more specifically an implant of the present invention comprises about 5% by weight of a polymer, or about 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59 60, 61, 62, 63, 64, 65, 66, 67, 68, 69, 70, 71, 72, 73, 74, 75, 76, 77, 78, 79, 80, 81, 82, 83, 84, 85 , 86, 87, 88, 89, or about 90% by weight of a biodegradable polymer or polymer combination. The polymer is selected from the group consisting of polylactic acid (PLA), polyglycolic acid (PGA), and polylactic acid polyglycolic acid (PLGA) copolymer. Combinations of any of the foregoing may also be used in a suitable ratio to prepare an implant of the invention with the final weight percentage of polymer given above. For example, an implant may comprise about 10-60% by weight of a poly (D,L-lactide) and about 5-20% by weight of a poly (D,L lactide-co-glycolide) copolymer.

In addition to siRNA and one or more biodegradable polymers, an implant of the present invention, including any of those described herein above, may comprise up to about 15 % (w/w) excipient, or from about 5 to about 10% (w/w) excipient. More specifically, an implant of the present invention can comprise about 1, 2, 3, 4, 5, 6, 7, 8, 9 , 10, 11, 12, 13, 14, or about 15% (w/w) excipient, wherein the excipient is any of those described herein for retarding the release of an siRNA from the implant relative to the implant without the excipient.

An implant of the present invention as described herein may release an amount of siRNA (e.g., a percentage of the initial load of siRNA) for at least about 1 day, 2 days, 3 days, 4 days, 5 days, 6 days, 7 days, 8 days, 9 days, 10 days, 11 days, 12 days, 13 days, or at least about 14 days after placement in the eye of a human or non-human subject. An implant according to the present invention, and according to any of the embodiments set forth above, may also release an amount of siRNA for at least about 1 week (7 days), 2 weeks, 3 weeks, 4 weeks, 5 weeks, 6 weeks, 7 weeks, or 8 weeks. For instance, an implant according to the present invention may release between about 2% and about 10% of the initial load of siRNA over a period of about 1 day. Additionally, an implant according to the present invention may be capable of releasing an amount of siRNA for at least about 1 month, 2 months, 3 months, 4 months, 5 months, or at least about 6 months, or between about 2 and about 6 months. In some instances, an implant may release an amount of siRNA for up to one year or more.

In other embodiments, implants disclosed herein may be configured such that the amount of the siRNA that is released from the implant within two days of being placed in the eye is less than about 95% of the total amount of the siRNA in the implant. In certain implants, 95% of the siRNA is not released until after about one week of being placed in an eye. In certain implants, about 50% of the siRNA is released within about one day of placement in the eye, and about 2% is released for about 1 month after being placed in the eye. In other implants, about 50% of the siRNA is released within about one day of placement in the eye, and about 1% is released for about 2 months after being placed in the eye.

The present invention is also directed to a biodegrable intraocular implant for the extended release of an siRNA, the implant comprising, consisting of, or consisting essentially of one or more biodegradable polymers, an siRNA, and an excipient, wherein the one or more biodegradable polymers is selected from the group consisting of polylactic acid, polyglycolic acid, polylactic acid polyglycolic acid copolymer (PLGA), wherein the excipient is selected from polylysine and spermidine, whereby the implant provides extended release of the siRNA in an eye of an individual when placed in the eye of an individual. In this embodiment, the biodegradable intraocular implant can comprise 5 to 40% by weight siRNA, 1 to 15% by weight excipient, 10 to 60% by weight poly (D,L-lactide) and 5 to 40% by weight of a 75:25 poly (D,L lactide-co-glycolide) copolymer. In a more specific embodiment, the biodegradable intraocular implant comprises about 5% by weight of the implant.

Also within the scope of the present invention is a biodegradable intraocular implant (implant 1) comprising about 5 to about 40% by weight of a small interfering RNA (siRNA), a biocompatible polymer matrix configured to release said siRNA into the eye of a patient at therapeutic levels for a time sufficient to treat an ocular condition or disease, and an amount of excipient effective to retard the initial burst release of siRNA from said implant (implant 1), as compared to the initial burst release of siRNA from a control implant, the control implant consisting of the biocompatible polymer matrix present in implant 1 and an amount of said siRNA equivalent to the amount of said siRNA present in implant 1. Said biocompatible polymer matrix comprises a polylactic acid (PLA), a polyglycolic acid (PGA), a polylactic acid polyglycolic acid (PLGA) copolymer, a mixture of PLA polymers, or a mixture of a PLA polymer and PLGA copolymer. Said excipient is selected from polylysine and spermidine. The excipient in said biodegradable intraocular implant can be present in the implant in an amount of about 1% to about 15% by weight of the implant, or in an amount of about 5% by weight of the implant. In particular embodiments according to the present disclosure, the biodegradable intraocular implant can comprise about 10-60% by weight poly (D,L-lactide) polymer R203S, about 5-20% by weight poly (D,L-lactide) polymer R202H, about 5-40% by weight poly (D,L lactide-co-glycolide) copolymer RG752S, and about 1-15% by weight polyethylene glycol 3350 (PEG 3350). More particularly, the biodegradable intraocular implant according to the present disclosure may consist essentially of about 5-40% by weight siRNA, about 10-60% by weight poly (D,L-lactide) polymer R203S, about 5-20% by weight poly (D,L-lactide) polymer R202H, about 5-40% by weight poly (D,L lactide-co-glycolide) copolymer RG752S, and about 1-15% by weight polyethylene glycol 3350 (PEG 3350), wherein the implant releases an amount of siRNA for more than one week after implantation into an eye. In a further embodiment according to the present disclosure, the biodegradable intraocular implant comprises or consists of about 30% by weight siRNA, about 45% by weight poly (D,L-lactide) polymer R203S, about 20% by weight poly (D,L-lactide) polymer R202H, and about 5% by weight polyethylene glycol 3350 (PEG 3350). In yet a more specific embodiment according to the present invention, the biodegradable intraocular implant comprises about 14% by weight siRNA, about 81 % by weight polylactic acid polyglycolic acid (PLGA) copolymer, and about 5% by weight excipient, said excipient selected from the group consisting of polylysine and spermidine.

The present invention is also directed to a biodegradable intraocular implant for the extended release of an siRNA, the implant comprising one or more biodegradable polymers, an siRNA, and an excipient, wherein the one or more biodegradable polymers is selected from the group consisting of polylactic acid, polyglycolic acid, polylactic acid polyglycolic acid copolymer (PLGA), and mixtures thereof, wherein the excipient is selected from the group consisting of polylysine and spermidine, whereby the implant provides extended release of the siRNA in an eye of an individual when placed in the eye of an individual. The biodegradable intraocular implant can comprise about 5 to 40% by weight siRNA, about 1 to 15% by weight excipient, about 10 to 60% by weight poly (D,L-lactide) and about 5 to 40% by weight of a 75:25 poly (D,L lactide-co-glycolide) copolymer. In one embodiment of the extended release implant, the excipient is present in an amount of about 5% by weight of the implant. In one embodiment, the biodegradable intraocular implant comprises a polylactic acid polyglycolic acid (PLGA) copolymer, and the siRNA is present in an amount of about 14% by weight of the implant, and the excipient is present in an amount of about 5% by weight of the implant. In a more specific embodiment, the implant comprises about 81 % by weight of the PLGA copolymer, about 14% by weight siRNA, and about 5% be weight excipient, wherein the excipient decreases or retards the initial rate of release of the siRNA from the implant as compared to the implant without excipient.

The following U.S. Patent Application Publications provide further information relating to the present disclosure:
U.S. Patent Application Publication 2009/0258924 discloses biocompatible sustained-release intraocular drug delivery systems comprising an siRNA, a polymeric component, and a release modifying excipient, such as a fatty alcohol, glycol, or polysaccharide.
U.S. Patent Application Publication 2009/0226531 discloses nanoparticles encapsulating siRNA that may be placed in the eye to treat or reduce the occurrence of one or more ocular conditions.
U.S. Patent Application Publication 2008/0107694 discloses a biocompatible sustained release intraocular drug delivery system comprising a protein or polynucleotide therapeutic agent, a polymeric carrier, and a long chain fatty alcohol release modifier.

The following examples are intended to illustrate the present invention. Example 1 is provided for information only. The data in Example 2 wherein the excipient is neither spermidine nor polylysine are provided for information only.

### Example 1

### Measuring the initial burst release of siRNA from an implant

The initial burst release of siRNA from a biodegradable implant was assayed *in vitro* as follows: siRNA implants were prepared and then placed in phosphate buffered saline (PBS), 0.01 M, pH 7.4 (release medium) at 37°C in a shaking water bath. At the desired time (e.g., 24 hrs after placement in the release medium), the implant-containing PBS solution was sampled. The amount of siRNA in a sample was quantified by reverse phase HPLC.

To study the effect of siRNA load on the initial burst release of siRNA from an implant, we prepared implants containing increasing amounts of an siRNA (siRNA 027) in a PLGA polymer, (i.e., the Boehringer Ingelheim Resomer RG752S). As shown in Figure 1, increasing the load of siRNA from 1% to 10% (w/w) in the implant increased the initial burst release of siRNA on day one from about 5% to about 40%.

### Example 2

### Controlling the initial burst release of siRNA from an implant

The initial burst release of siRNA from implants can be retarded by the selective use of certain pharmaceutical excipients. To explore the effect of various excipients on the initial burst release of siRNA from an implant, a series of implants with different excipients were prepared and then evaluated as described in Example 1. Each implant contained 5% (w/w) excipient, 14% (w/w) siRNA-027, and 81% (w/w) PLGA polymer RG752S. The excipients tested are set forth in Figure 2. As shown in Figure 2, the inclusion of certain excipients in a biodegradable polymeric matrix (such as one comprising PLGA) significantly reduces the initial burst release of siRNA from the implant, as compared to implants without excipient. As compared to an implant having no excipient and consisting essentially of siRNA and a biodegradable polymer, as shown in Figure 1, the inclusion of excipient (shown in Figure 2) reduced the initial day one release of siRNA from the implant from over 40 % to about 2 to 10 %. Some excipients, such as sodium alginate and HPMC did not retard siRNA release.

In addition to retarding the initial rate of release of siRNA from the implant, certain excipients, such as those that form charge-charge complexes with the siRNA, may also facilitate the uptake of the siRNA by cells. For example, cationic molecules such as polylysine and spermidine in addition to retarding the initial burst release of siRNA from the implant, as shown in Figure 1, could also potentially promote the cellular uptake of the siRNA once released from the implant. In this regard, the excipients such as polylysine and spermidine could provide for not only extended release of the siRNA but also potentially for enhanced transfection of the siRNA after release.

The present invention is not to be limited in scope by the exemplified embodiments, which are only intended as illustrations of specific aspects of the invention. Various modifications of the invention, in addition to those disclosed herein, will be apparent to those skilled in the art by a careful reading of the specification, including the claims, as originally filed.

## Claims

1. A biodegrable intraocular implant for the extended release of a small interfering RNA (siRNA), the implant comprising one or more biodegradable polymers, an siRNA, and an excipient, wherein the one or more biodegradable polymers is selected from the group consisting of polylactic acid, polyglycolic acid, polylactic acid polyglycolic acid copolymer (PLGA), and mixtures thereof, wherein the excipient is selected from polylysine and spermidine, whereby the implant provides extended release of the siRNA in an eye of an individual when placed in the eye of an individual.

2. The biodegradable intraocular implant of claim 1, comprising 5 to 40% by weight siRNA, 1 to 15% by weight excipient, 10 to 60% by weight poly (D,L-lactide) and 5 to 40% by weight of a 75:25 poly (D,L lactide-co-glycolide) copolymer.

3. The biodegradable intraocular implant of claim 1, wherein the excipient is present in an amount of 5% by weight of the implant.

4. The biodegradable intraocular implant of claim 1, comprising a polylactic acid polyglycolic acid (PLGA) copolymer, wherein the siRNA is present in an amount of 14% by weight of the implant, and wherein the excipient is present in an amount of 5% by weight of the implant.

5. The biodegradable intraocular implant of claim 4, wherein the implant comprises 81% by weight of the PLGA copolymer.

6. A biodegradable intraocular implant according to claim 1 comprising 5 to 40% by weight of an siRNA, a biocompatible polymer matrix configured to release said siRNA into the eye of a patient at therapeutic levels for a time sufficient to treat an ocular condition or disease, and an excipient, wherein said biocompatible polymer matrix comprises a polylactic acid (PLA), a polyglycolic acid (PGA), a polylactic acid polyglycolic acid (PLGA) copolymer, a mixture of PLA polymers, or a mixture of a PLA polymer and PLGA copolymer, wherein said excipient is selected from polylysine, and spermidine, wherein said excipient is present in the implant in an amount of 1% to 15% by weight of the implant.

7. The implant of claim 6, wherein said excipient is present in the implant in an amount of 5% by weight of the implant.

8. The biodegradable intraocular implant of claim 6 comprising 14% by weight siRNA, 81% by weight polylactic acid polyglycolic acid (PLGA) copolymer, and 5% by weight excipient, said excipient selected from polylysine and spermidine.

## Patentansprüche

1. Bioabbaubares intraokulares Implantat für die verlängerte Freisetzung einer kleinen interferierenden RNA (siRNA), wobei das Implantat ein oder mehrere bioabbaubare Polymere, eine siRNA und einen Hilfsstoff umfasst, wobei das eine oder die mehreren bioabbaubare(n) Polymer(e) ausgewählt wird (werden) aus der Gruppe, bestehend aus Polymilchsäure, Polyglycolsäure, Polymilchsäure-Polyglycolsäure-Copolymer (PLGA) und Mischungen davon, wobei der Hilfsstoff ausgewählt wird aus Polylysin und Spermidin, wobei das Implantat verlängerte Freisetzung der siRNA in einem Auge eines Individuums bereitstellt, wenn es im Auge des Individuums platziert wird.

2. Bioabbaubares intraokulares Implantat nach Anspruch 1, umfassend 5 bis 40 Gew.-% siRNA, 1 bis 15 Gew.-% Hilfsstoff, 10 bis 60 Gew.-% Poly(D,L-Lactid) und 5 bis 40 Gew.-% eines 75:25 Poly(D,L-Lactid-Co-Glycolid)-Copolymers.

3. Bioabbaubares intraokulares Implantat nach Anspruch 1, wobei der Hilfsstoff in einer Menge von 5 Gew.-% bezogen auf das Implantat vorliegt.

4. Bioabbaubares intraokulares Implantat nach Anspruch 1, umfassend ein Polymilchsäure-Polyglycolsäure- (PLGA-) Copolymer, wobei die siRNA in einer Menge von 14 Gew.-% bezogen auf das Implantat vorliegt und wobei der Hilfsstoff in einer Menge von 5 Gew.-% bezogen auf das Implantat vorliegt.

5. Bioabbaubares intraokulares Implantat gemäß Anspruch 4, wobei das Implantat 81 Gew.-% des PLGA-Copolymers umfasst.

6. Bioabbaubares intraokulares Implantat gemäß Anspruch 1, umfassend 5 bis 40 Gew.-% einer siRNA, eine biokompatible Polymermatrix, die zur Freisetzung der siRNA in das Auge eines Patienten in therapeutischen Konzentrationen über eine zum Behandeln eines Augenzustands oder eines Augenleidens ausreichende Zeit konfiguriert ist, und einen Hilfsstoff, wobei die biokompatible Polymermatrix eine Polymilchsäure (PLA), eine Polyglycolsäure (PGA), ein Polymilchsäure-Polyglycolsäure- (PLGA-) Copolymer, eine Mischung aus PLA-Polymeren oder eine Mischung aus einem PLA-Polymer und einem PLGA-Copolymer umfasst, wobei der Hilfsstoff ausgewählt wird aus Polylysin und Spermidin, wobei der Hilfsstoff in einer Menge von 1 Gew.-% bis 15 Gew.-% bezogen auf das Implantat vorliegt.

7. Implantat gemäß Anspruch 6, wobei der Hilfsstoff in dem Implantat in einer Menge von 5 Gew.-% bezogen auf das Implantat vorliegt.

8. Bioabbaubares intraokulares Implantat nach Anspruch 6, umfassend 14 Gew.-% siRNA, 81 Gew.-% Polymilchsäure-Polyglycolsäure- (PLGA-) Copolymer und 5 Gew.-% Hilfsstoff, wobei der Hilfsstoff ausgewählt wird aus Polylysin und Spermidin.

## Revendications

1. Implant intraoculaire biodégradable pour la libération prolongée d'un petit ARN interférent (ARNsi), l'implant comprenant un ou plusieurs polymères biodégradables, un ARNsi et un excipient, dans lequel lesdits un ou plus de polymères biodégradables sont choisis dans le groupe constitué de l'acide polylactique, de l'acide polyglycolique, d'un copolymère d'acide polylactique et d'acide glycolique (PLGA) et des mélanges de ceux-ci, dans lequel l'excipient est choisi parmi la polylysine et la spermidine, dans lequel l'implant permet une libération prolongée de l'ARNsi dans un oeil d'un individu lorsqu'il est placé dans l'oeil d'un individu.

2. Implant intraoculaire biodégradable selon la revendication 1, comprenant 5 à 40 % en poids d'ARNsi, 1 à 15 % en poids d'excipient, 10 à 60 % en poids de poly(D,L-lactide) et 5 à 40 % en poids d'un copolymère à 75:25 de poly(D,L-lactide-co-glycolide).

3. Implant intraoculaire biodégradable selon la revendication 1, dans lequel l'excipient est présent en quantité de 5 % en poids de l'implant.

4. Implant intraoculaire biodégradable selon la revendication 1, comprenant un copolymère d'acide polylactique et d'acide polyglycolique (PLGA), dans lequel l'ARNsi est présent en quantité de 14 % en poids de l'implant et dans lequel l'excipient est présent en quantité de 5 % en poids de l'implant.

5. Implant intraoculaire biodégradable selon la revendication 4, dans lequel l'implant comprend 81 % en poids du copolymère de PLGA.

6. Implant intraoculaire biodégradable selon la revendication 1, comprenant 5 à 40 % en poids d'un ARNsi, une matrice polymère biocompatible configurée pour libérer ledit ARNsi dans l'oeil d'un patient à des niveaux thérapeutiques pendant une période de temps suffisante pour traiter une pathologie ou une maladie oculaire, et un excipient, dans lequel ladite matrice polymère biocompatible comprend un acide polylactique (PLA), un acide polyglycolique (PGA), un copolymère d'acide polylactique et d'acide polyglycolique (PLGA), un mélange de polymères de PLA ou un mélange d'un polymère de PLA et d'un copolymère de PLGA, dans lequel ledit excipient est choisi parmi la polylysine et la spermidine, dans lequel ledit excipient est présent dans l'implant en quantité de 1 % à 15 % en poids de l'implant.

7. Implant selon la revendication 6, dans lequel ledit excipient est présent dans l'implant en quantité de 5 % en poids de l'implant.

8. Implant intraoculaire biodégradable selon la revendication 6, comprenant 14 % en poids d'ARNsi, 81 % en poids d'un copolymère d'acide polylactique et d'acide polyglycolique (PLGA) et 5 % en poids d'excipient, ledit excipient étant choisi parmi la polylysine et la spermidine.
